# EUROPEAN PATENT SPECIFICATION

(11) **EP 4 137 052 B1**
(45) Date of publication and mention of the grant of the patent: **13.03.2024**
(21) Application number: 21192435.2
(22) Date of filing: 20.08.2021
(51) Int. Cl.: A61B 5/35, A61B 5/361, A61B 5/00, G16H 50/20

(54) **METHOD FOR DETECTING A CARDIAC ISOLATION STATUS OF A MEASUREMENT LOCATION IN THE PRESENCE OF FAR FIELD INTERFERENCE**
VERFAHREN ZUR ERKENNUNG EINES HERZISOLATIONSZUSTANDES EINER MESSSTELLE IN PRÄSENZ VON FERNFELDSTÖRUNGEN
PROCÉDÉ DE DÉTECTION DE L'ÉTAT D'ISOLATION CARDIAQUE D'UN EMPLACEMENT DE MESURE EN PRÉSENCE D'INTERFÉRENCE DE CHAMP LOINTAIN

(43) Date of publication of application: 22.02.2023
(73) Proprietor: CathVision ApS, 2200 Copenhagen (DK)
(72) Inventor: PAAMAND, Rune, 3060 Humlebaek (DK); MATTHIESEN, Mads Emil, 24771 Genarp (SE)
(74) Representative: Gottschald Patentanwälte Partnerschaft mbB

(56) References cited:
- EP-A1- 3 451 917
- EP-B1- 3 451 917
- US-A1- 2002 019 593

## Description

The invention relates to a method for determining a cardiac isolation status of a measurement location in the presence of far field interference according to claim 1 and to a control system adapted to execute the method according to claim 15.

The present method is particularly concerned with atrial fibrillation and atrial flutter. Electrically, atrial fibrillation is chaotic activation of muscle cells of the atrial. During atrial fibrillation, the atria only minimally contribute to the function of the heart. Atrial fibrillation, therefore, reduces the output of the heart but is not imminently dangerous. However, when becoming chronical, atrial fibrillation is correlated width increased morbidity and mortality. One treatment option for atrial fibrillation is ablation therapy. Ablation is the destruction of the cells that allow electrical wave re-entry to reduce chaotic activation of the atrial muscle cells.

A recommended treatment for atrial fibrillation includes pulmonary vein isolation. Pulmonary vein isolation can be performed with various ablation techniques, including radio frequency ablation, cryo balloon ablation and pulsed-field ablation. While these techniques apply energy differently, their common endpoint is the isolation of the electrical activity of the pulmonary veins from the rest of the atrium.

Generally, ablation therapy is successful, if the targeted location is electrically isolated from the rest of the heart. There are different methods for assessing the success of the ablation therapy. These include costly 3D mapping of ablation points and measurements of force or temperature during ablation and electrogram analysis.

One known method (EP 3 139 828 B1) focusses on a morphology analysis of local activation potentials of a measurement location. In this method, the local activation potentials are classified into groups according to the number and characteristics of their peaks. An analysis of the distribution of the potentials across the morphological groups allows a good classification of a cardiac isolation status of the measurement location. A method based on electrical signals is cheap in the implementation and easily usable. Further, measuring electrical waves targets the actual physical effect underlying atrial fibrillation rather than a secondary factor.

The known method provides good results. However, in the known method the local activation potentials of the measurement location are detected based on the detection of a reference CS-potential. This CS-potential is detected by a coronary sinus catheter. Based on this reference CS-potential, a predetermined window of approximately 200ms is chosen. This window is defined a s containing the local activation potentials of the channels related to the measurement location. Therefore, the reliability of the known method depends on the patient being in a sinus rhythm.

Further methods are known from US 2002/019593 A1, which discloses a method of classifying cardiac complexes to determine different states of tachycardia, and EP 3 451 917 A1, which discloses a cardiac mapping system.

There exists a demand for a method for determining an isolation status using electrograms and analysis of local activation potentials usable without a-priori knowledge about the location of the local activation potentials from a reference potential. This is necessary for example if a cardiac isolation status of a patient should be determined while the patient is in atrial fibrillation. During atrial fibrillation, the time relation between the CS-potential and local activations may be broken. While the possibility to cardiovert a patient to a normal sinus rhythm exists, this may not always be desired.

It is further problematic that cardiac electrograms contain not only local activation potentials but also far field interference. Measurement of the CS-potential also typically requires a separate CS-catheter in addition to a catheter that may be located in the pulmonary vein. It is desirable to identify methods for determining cardiac isolation status that can also be performed with a single catheter.

It is therefore an object of the present invention to provide a method for detecting a cardiac isolation status in the presence of far field interference usable without being able to rely on a reference CS-potential for detecting local activations.

The above-noted object is solved by the method of claim 1.

The main realization of the present invention is that by analyzing multiple channels of an intracardiac electrogram independently and searching for local activation potentials on the channels without using a single resulting time window on all channels, sufficient actual local activation potentials can be found even during atrial fibrillation to enable analyzing those local activation potentials to determine a cardiac isolation status of the measurement location.

In particular, this enables determining a cardiac isolation status of a measurement location during atrial fibrillation.

In detail, a method for determining a cardiac isolation status of a measurement location in the presence of far field interference by analyzing a multi-channel intracardiac electrogram of the measurement location via a control system, wherein in an identification routine the control system applies an activation search algorithm to analysis windows of at least 400 ms in at least two different channels of the intracardiac electrogram, wherein the activation search algorithm identifies windows of local activation potentials inside of the analysis windows, wherein in a classification routine the control system analyzes the local activation potentials to determine the cardiac isolation status of the measurement location, is proposed.

Claim 2 specifies the very preferred applications of the proposed method. In particular during atrial fibrillation or atrial flutter, local activation potentials, particularly pulmonary vein local activation potentials, cannot be detected by relying on a-priori-knowledge about their position in time from a reference CS-potential.

In an embodiment according to claim 3, the control system analyses in the classification routine a morphology of the local activation potentials to determine a cardiac isolation status. It has been realized that a morphology classification of local activation potentials can be done effectively even during atrial fibrillation. Claim 4 specifies preferred details of the classification routine. In general, morphologies with less fractionated peaks, lower peak frequencies, lower peak sharpness and peaks of lesser amplitudes indicate a successful isolation. Claim 5 relates to different preferred morphology groups that have been shown to enable a successful isolation status determination.

The embodiments of claim 6 relate to preferred widths of the analysis windows chosen to ensure mostly having at least one physiological local activation potential in each analysis window. It is preferred to choose the width of the analysis window such that at least one physiological local activation potential is present in each analysis window. This allows adapting the activation search algorithm such that it can always find a local activation potential because the analysis window contains at least one. For the present algorithm it is not necessary to find all local activation potentials. Nevertheless, in an embodiment according to claim 7, sliding windows are used to find multiple local activation potentials per channel.

Claim 8 is concerned with the possibility of using the proposed method to verify the success of an ablation procedure. As an ablation procedure is an invasive surgical operation, observing a patient for days to determine the success of an ablation procedure is not preferred. Rather, the success of the ablation procedure should be determined during the intervention.

The activation search algorithm may comprise a peak detection algorithm described in claim 9.

The control system may identify a fixed number of local activation potentials per channel (claim 10). This number may be smaller or equal to the number of physiologically present local activation potentials. In this way the number of incorrectly identified local activation potentials can be reduced.

In a preferred embodiment according to claim 11, the control system may execute an interference signal removal step prior to the identification step, in particular to remove far field interference signals. Claim 11 names removal of signals around and/or relative to pacing artifacts, CS-potentials and ECG-waves. By removing time windows related to sources of far field interference, the chance of detecting actual local activation potentials is greatly increased. This removal is called blanking. Such blanking may also be done in a weighted manner to enable identifying local activation potentials that partly overlap with the blanked time frames. The blanking may for example be weighted by a gauss curve fully or almost fully removing the electrogram in the middle of the time window to be blanked and only reducing an amplitude at the edges. Further details of the weighted blanking are subject of claim 12.

To reduce a number of incorrectly identified local activation potentials, the control system may execute a quality control step according to claim 13. Sections of the electrogram or even channels of the electrogram may be removed from consideration before or after the identification step based on a quality parameter.

Claim 14 relates to preferably used catheters.

Another teaching according to claim 15, which is of equal importance, is directed to a control system configured to perform the proposed method. All explanations given with respect to the proposed method are fully applicable.

In the following, an embodiment of the invention is explained with respect to the drawings. The drawings show in
- fig. 1: the proposed control system during the measurement of a multi-channel intracardiac electrogram, and in
- fig. 2: the proposed method in an exemplary application.

The proposed method is used for determining a cardiac isolation status of a measurement location 1 in the presence of far field interference by analysing a multi-channel intracardiac electrogram 2 of the measurement location 1 via a control system 3. The cardiac isolation status may be a binary yes/no decision or a percentage of a probability of isolation of the measurement location 1. The multi-channel intracardiac electrogram 2 is preferably measured by an intracardiac catheter 4 comprising a number of electrodes 5, for example ten electrodes 5. These electrodes 5 have a certain spatial extension. Therefore, the measurement location 1 also has a certain spatial extension. It is not necessary however for all electrodes 5 of the measurement catheter 6 to be located at the measurement location 1. It might be the case that the measurement catheter 6 is placed across a boundary of an ablation lesion 7 for example.

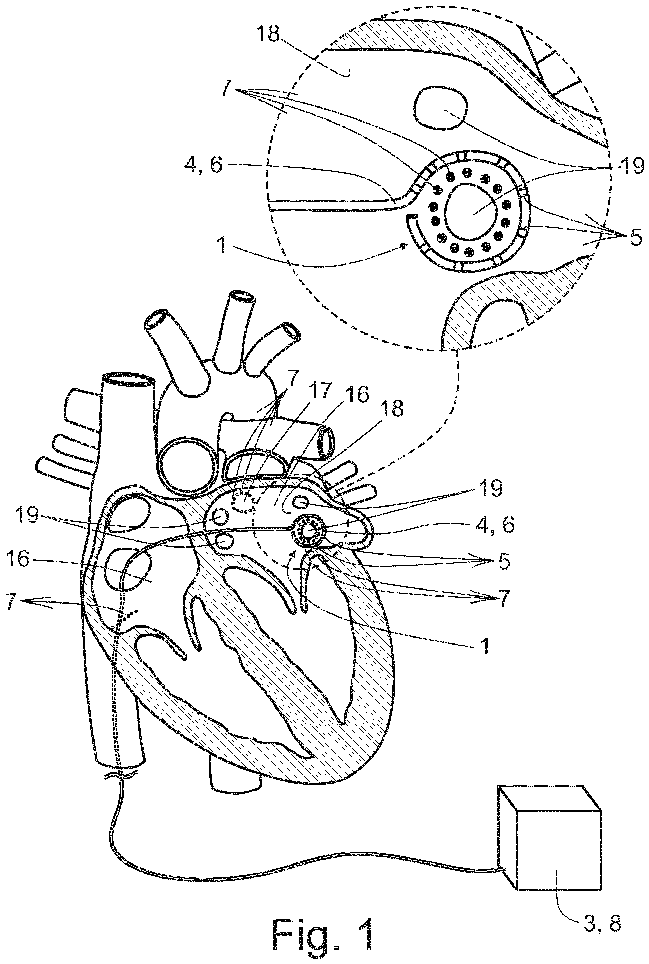

The control system 3 may be a local unit 8 with a processor, possibly a user interface and the like as shown in fig. 1. It may also comprise in one embodiment a cloud processor. It this therefore not necessary for the control system 3 to be confined to a single device.

In an identification routine 9 the control system 3 applies an activation search algorithm to analysis windows 10 of at least 400ms in at least two different channels 11 of the intracardiac electrogram 2. A channel 11 is defined as the measurement between at least two electrodes 5. The proposed method serves to determine a cardiac isolation status when local activation potentials 12 cannot be reliably located relative to a reference CS-potential 13. This is particularly true during atrial fibrillation.

The activation search algorithm identifies windows W of local activation potentials 12 inside of the analysis windows 10. It is essential that these windows W of local activation potentials 12 can differ regarding their location in time between channels 11 and are not set equal for all channels 11 relative to the timing of a reference potential 14. Therefore, the search field for these windows W is chosen to be at least 400 ms wide. In general, the windows W of the local activation potentials 12 should comprise all or most of the relevant waveform of the respective local activation potential 12. The activation search algorithm may be an algorithm searching directly for the window W or searching for a single point in time assigned to the local activation potential 12 and derive the window W from there.

By searching local activation potentials 12 without a reference timing, it becomes possible to determine a cardiac isolation status without knowledge about any reference timing.

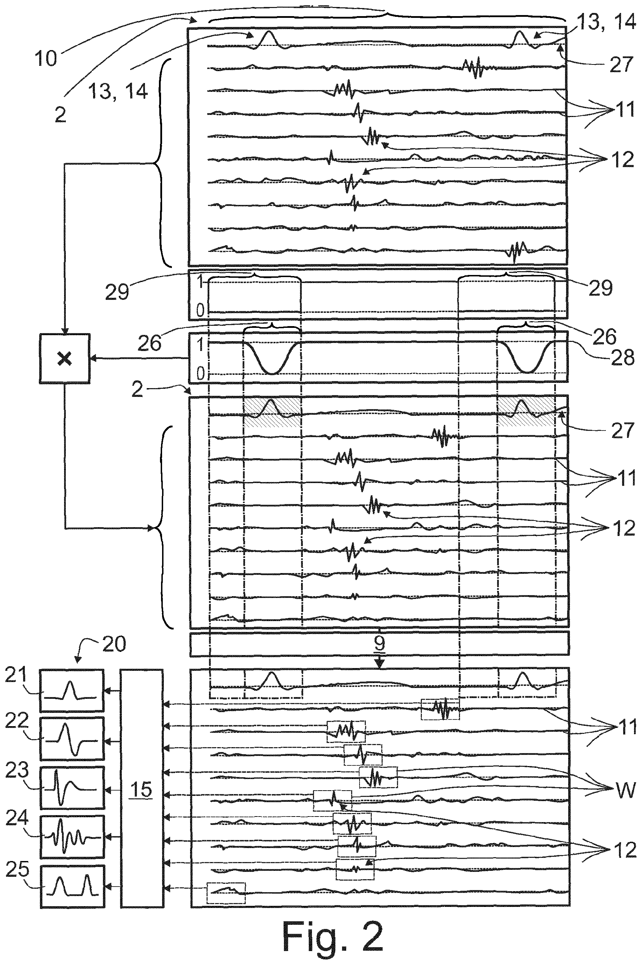

Fig. 2 shows the application of the identification routine 9 to a multi-channel intracardiac electrogram 2 and resulting windows W of local activation potentials 12. Here and preferably the windows W of the local activation potentials 12 have a width of at most 250 ms, preferably at most 200 ms, more preferably at most 175 ms and more preferably at most 150 ms. Here they have a width of approximately 128 ms. Preferably, the width of the windows W of local activation potentials 12 is at least 50 ms.

In a classification routine 15 the control system 3 analyses the local activation potential 12 to determine the cardiac isolation status of the measurement location 1. The classification routine 15 may use the algorithm of EP 3 139 828 B1.

As has already been described, it is preferred that the intracardiac electrogram 2 has been recorded during an atrial arrhythmia, in particular atrial fibrillation or atrial flutter. The proposed method allows determining the cardiac isolation status even in those cases.

As is schematically shown in fig. 1, the measurement location 1 may be at least partly located inside an atrium 16, in particular inside the left atrium 16. The measurement location 1 may be an isle 17 in the wall 18 of the left atrium 16 or at the entrance of a pulmonary vein 19. There are different locations that can be targeted with ablation. A main target are the entrances of the pulmonary veins 19 into the left atrium 16. Successfully isolating the pulmonary veins 19 may be the target of an ablation therapy. Therefore the measurement location 1 may be at least partly located inside a pulmonary vein 19.

In general, the proposed method can be used prior to, during and/or after an ablation therapy. It can be used to determine an isolation status of a potential target for ablation in which case the isolation status may comprise information about how the measurement location 1 is involved with the conduction of electrical activations. Here and preferably the cardiac isolation status contains information about how well an ablation therapy has isolated the measurement location 1 from a remainder of the heart. Here and preferably the proposed method is used during and or after an ablation therapy. The measurement location 1 may then be the target of the ongoing or concluded ablation therapy.

Turning now to the classification routine 15, it may be the case that in the classification routine 15 the control system 3 analyses a morphology of the local activation potentials 12 to determine the cardiac isolation status of the measurement location 1. It has been found that information about the isolation status is contained in the complexity and amplitude of the local activation potentials 12. The morphology of the local activation potentials 12 depends on whether these are caused by propagation of a more global activation of the heart or a local pacing event and whether the general electrical situation is chaotic as in atrial fibrillation.

In the classification routine 15 the control system 3 may classify the local activation potential 12 into morphology groups 20 and preferably determines based on the distribution of local activation potentials 12 across the groups the cardiac isolation status. It is particularly advantageous that incorrectly identified local activation potentials 12, which may be caused by noise or far field interference, often show a morphology distinct from the morphology of local activation potentials 12 from a non-isolated location or an isolated location, allowing to ignore these false detections.

The control system 3 may classify local activation potentials 12 into morphology groups 20 based on a number of characteristic peaks of the local activation potential 12. For this, not every plateau in the electrogram 2 necessarily counts as a characteristic peak. Preferably, the control system 3 classifies a peak with at least a predetermined amplitude and/or with at least a predetermined slope and/or with at most a predetermined slope and/or with at least a predetermined minimum peak distance and/or with at most a predetermined maximum peak distance and/or based on a peak morphology, in particular a minimum and/or maximum peak angle, as a characteristic peak. Classification as a characteristic peak can also be based on the peaks having a predetermined peak frequency and/or a predetermined peak sharpness.

Here and preferably the morphology groups 20 comprise a group for local activation potentials 12 with a single characteristic peak, "monophasic 21", and/or a group for local activation potentials 12 with exactly two characteristic peaks, "biphasic 22" and/or exactly three characteristic peaks, "triphasic 23", and/or more than three characteristic peaks, "multiphasic 24". The morphology groups 20 may further or alternatively comprise a group for local activation potentials 12 with at least two characteristic peaks separated by a predetermined time, "double potentials 25". They may further comprise a group for local activation potentials 12 that show a morphology associated with erroneously detected local activation potentials 12 either stemming from noise or far field interference for example.

It is preferred to chose the analysis windows 10 such that at least one physiological local activation potential 12 is probably or surely present. For this, the analysis windows 10 may have a width of at least 400 ms, preferably at least 800 ms, more preferably at least 1,25 s. In a preferred embodiment, the analysis windows 10 may have a width of at most 3 s, preferably at most 2 s, more preferably at most 1,75 s. Here the analysis windows 10 have a width of 1,5 s.

It is preferred to extract multiple local activation potentials 12 from each channel 11 to enable a good statistical analysis of the local activation potentials 12, in particular of their morphology groups 20. Here and preferably in total at least 2, preferably at least 5, in particular at most 10, local activation potentials 12 are extracted. To extract multiple local activation potentials 12 per channel, the analysis windows 10 may be overlapping or non-overlapping sliding windows over a measurement time for each channel. Preferably at least two local activation potentials 12 are extracted per channel 11 whereby in particular at least one local activation potential 12 may be extracted per analysis window 10. The measurement time may be chosen to be at least 1 s, preferably at least 2,5 s, more preferably at least 5 s. The measurement time may be chosen to be at most 10 s.

As mentioned above, the control system 3 may carry out the identification routine 9 and the classification routine 15 on a multi-channel intracardiac electrogram 2 of the measurement location 1 recorded after an ablation procedure applied near to, in particular around, the measurement location 1 to determine the cardiac isolation status of the measurement location 1. The control system 3 may thereby evaluate the success of the ablation procedure.

It may be preferred to compare data before and after the ablation procedure. Therefore, it may be the case that additionally the control system 3 carries out at least the identification routine 9 on a multi-channel intracardiac electrogram 2 of the measurement location 1 recorded prior to the ablation procedure and determines the cardiac isolation status based on a comparison of the local activation potentials 12 prior to and after the ablation procedure. The measurement locations 1 prior to and after the ablation procedure do not have to be identical, a reasonable co-incidence may suffice.

The activation search algorithm may comprise a peak detection algorithm for finding the local activation potentials 12 and subsequently the windows W of the local activation potentials 12. The peak detection algorithm may be based on non-linear filters, in particular wavelet filters, and/or it may be based on a transformation of the electrogram 2, in particular a wavelet transformation. The peak detection algorithm may comprise a peak detection by an amplitude. For example, the highest amplitude inside of a given time frame may be detected as a peak.

Returning to the detection of local activation potentials 12 by taking into consideration their frequency under physiological conditions, in the identification routine 9 the control system 3 may identify a fixed number of local activation potentials 12 per channel 11 and/or per analysis window 10. Preferably the control system 3 in the identification routine 9 may identify a fixed number of local activation potentials 12 per time interval. This time interval may be the measurement window. The fixed number may be based on a physiological and/or measured heartrate. It may be the case that the fixed number is a maximum of one local activation potential 12 per at least 500 ms, preferably per at least 800 ms, more preferably per at least 1 s. By detecting only a single local activation potential 12 inside of any given time window, for example 800 ms, it is possible to ensure that the activation search algorithm always has the possibility of finding a real local activation potential 12.

With regards to fig. 2 the removal of interference signals, in particular from far field interference, will now be described in more detail. The control system 3 may execute an interference signal removal step prior to the identification step. The interference signal removal step may comprise complete or weighted blanking of time intervals 26 around and/or relative to pacing artefacts and or CS-potentials 13 and or ECG-waves 27. ECG-waves 27 may comprise P-waves, QRS-complexes and/or T-waves, that can be associated with atrial and ventricular activity. Generally, other artefacts may be blanked too. This blanking in the weighted form is shown in fig. 2.

In a simple embodiment, the blanking can be seen as removing part of the electrogram 2 or multiplying the respective part of the electrogram 2 with zero. Here and preferably the blanking is applied to all channels 11. In the weighted form, the blanked time interval 26 of the electrogram 2 is not completely multiplied with zero. It may be only reduced in amplitude, in particular at the edges and near the edges of the time interval 26, where the far field interference is expected to be moderate. This makes it possible to find local activation potentials 12 partly overlapping with the blanked time interval 26.

The pacing artefacts and/or the CS-potentials 13 and or the ECG-waves 27 may be detected on an electrogram 2 different from the multi-channel intracardiac electrogram 2, the electrogram 2 be a coronary sinus or a surface electrogram 2. These are generally more suited to identify for example CS-potentials 13 and ECG-waves 27, such as P-waves, QRS-complexes and T-waves, associated with the atrial and ventricular activity, than a potentially isolated pulmonary vein 19 electrogram 2.

The CS-potentials 13 and ECG-waves 27 may be detected by known algorithms, for example by using peak detection on the surface electrogram 2. The pacing artefacts can be detected through peak amplitude detection and/or slope analysis.

As part of the interference signal removal step or as an independent step, a classification of interference signals may be done and may be used for blanking said interference signals. Here and preferably, the interference signals are classified as at least one of pacing artifacts and/or far field interference and/or instable activation potentials and/or noisy activation potentials. Detected interference signals may be excluded from being identified as local activation potentials 12.

Pacing artifacts are signals generated by methods of cardiac pacing. The pacing artifacts may be classified by analysing voltage characteristics, in particular a slope of the channels 11. Local activation potentials 12 show a physiological delay between channels 11 respectively the real locations associated with the channels. Therefore, the control system 3 may classify signals that occur inside of a predetermined short time window within many or all channels 11 as pacing artifacts. Alternatively, the control system 3 may comprise an input for external pacing timing.

The far field interference may be classified by determining the timing of the QRS activations from a surface ECG, for example through delineation of the ECG in P-waves, QRS-complexes and/or T-waves, wherein each wave may be associated with specific atrial or ventricular activity.

Instable activation potentials may be such activation potentials that have an energy which is not well localized, have baseline deviations and/or large wave-to-wave amplitude deviations. These instable activation potentials may be excluded from further analysis, in particular by blanking. The instable activation potentials can be classified based on expected waveforms of the surface ECG and/or the intracardiac electrogram 2. Alternatively, instable activation potentials can be identified in the classification routine 15.

Noisy activation potentials are such activation potentials where the noise spectrum overlaps the physiological spectrum in a relevant manner. Known methods for noise estimation can be used. Here and preferably the weighted blanking is an application of different weights 28 to the electrogram 2 inside the respective time interval 26. Preferably the weights 28 are predetermined to comprise a section of complete removal of the respective time interval 26, for example a multiplication with zero, and at least one section of reducing the amplitude of the electrogram 2. The weighted blanking can be applied by other methods than multiplication. An example of weights 28 may be a gauss curve.

The weighted blanking can be parameterized, in particular regarding the weights and/or the length of the blanking, based on the classification of the interference signal that is to be blanked. This way it becomes possible to detect local activation potentials 12 that have a high probability of showing a clear signal even during atrial fibrillation thereby allowing the analysis of local activation potentials during atrial fibrillation.

As an example, a pacing artifact can be entirely suppressed from the onset and a defined period following the onset after which the blanking is reduced. The length and/or reduction may be specific to hardware or software filter settings used with the control system 3.

A QRS complex may be dynamically penalized estimated from the associated influence on the last few beats. The present approach allows to track a local activation potential 12 that gradually delays and overlaps the QRS timing, which happens often at the critical moment of isolation. Therefore, the present approach allows determining the cardiac isolation status during an isolation procedure and during atrial fibrillation without the necessity to cardiovert the patient to a normal sinus rhythm.

Fig. 2 shows another example for a blanking of a time interval 29, in this case relative to the CS-potentials 13. Here, a complete blanking of the signal in time intervals 29 may be performed by multiplication with zero.

The interference signal removal step, in particular by means of blanking, may be performed with regard to the identification step on the intracardiac electrogram 2, while the classification routine 15 may be based on an analysis of the local activation potentials 12 in the intracardiac electrogram 2 without or a different type of interference signal removal as used for the identification.

The control system 3 may further execute a quality control step. In the quality control step, the control system 3 removes local activation potentials 12 and/or time sections of electrogram 2 channels 11 and/or electrogram 2 channels 11 as such, based on a quality parameter. The quality parameter may be a noise parameter, preferably a root-mean-square ratio and/or based on a power-line interference detection and/or the noise parameter may be a peak-to-baseline ratio.

With regards to fig. 1, the multi-channel electrogram 2 may comprise at least four, preferably at least six, more preferably at least eight channels 11. The multi-channel electrogram 2 may have been recorded by an ablation catheter 6. Other possibilities are a circular catheter 6 or a multi-spline catheter 6 for example. The multi-channel electrogram 2 may be a bipolar electrogram. In a preferred alternative, the multi-channel electrogram 2 is a unipolar electrogram.

According to another teaching, which is of equal importance, a control system 3 configured to perform the proposed method is proposed. All explanations given with regards to the proposed method are fully applicable. The control system 3 is configured to receive and/or measure the multi-channel electrogram 2. The control system 3 is preferably connectable to the ablation catheter 6.

## Claims

1. Method for determining a cardiac isolation status of a measurement location (1) in the presence of far field interference by analysing a multi-channel intracardiac electrogram (2) of the measurement location (1) via a control system (3),
wherein in an identification routine (9) the control system (3) applies an activation search algorithm to analysis windows (10) of at least 400 ms in at least two different channels (11) of the intracardiac electrogram (2), wherein the activation search algorithm identifies windows (W) of local activation potentials (12) inside of the analysis windows (10),
wherein in a classification routine (15) the control system (3) analyses the local activation potentials (12) to determine the cardiac isolation status of the measurement location (1).

2. Method according to claim 1, **characterized in that** the intracardiac electrogram (2) has been recorded during an atrial arrhythmia, in particular atrial fibrillation or atrial flutter, and/or, that the measurement location (1) is at least partly located inside an atrium (16), in particular the left atrium (16), preferably that the measurement location (1) is an isle (17) in the wall (18) of the left atrium (16) or at the entrance of a pulmonary vein (19), and/or, that the measurement location (1) is at least partly located inside a pulmonary vein (19).

3. Method according to claim 1 or 2, **characterized in that** in the classification routine (15) the control system (3) analyses a morphology of the local activation potentials (12) to determine the cardiac isolation status of the measurement location (1), preferably that in the classification routine (15) the control system (3) classifies the local activation potentials (12) into morphology groups (20) and preferably determines based on the distribution of local activation potentials (12) across the groups the cardiac isolation status.

4. Method according to claim 3, **characterized in that** in the classification routine (15) the control system (3) classifies the local activation potentials (12) into morphology groups (20) based on a number of characteristic peaks of the local activation potential (12), preferably that the control system (3) classifies a peak with at least a predetermined amplitude and/or with at least a predetermined slope and/or with at most a predetermined slope and/or with at least a predetermined minimum peak distance and/or with at most a predetermined maximum peak distance and/or based on a peak morphology, in particular a minimum and/or maximum peak angle, as a characteristic peak.

5. Method according to claim 4, **characterized in that** the morphology groups (20) comprise a group for local activation potentials (12) with a single characteristic peak and/or exactly two characteristic peaks and/or exactly three characteristic peaks and/or more than three characteristic peaks and/or at least two characteristic peaks separated by a predetermined time.

6. Method according to one of the preceding claims, **characterized in that** the analysis windows (10) have a width of at least 400 ms, preferably at least 800 ms, more preferably at least 1,25 s, and/or that the analysis windows (10) have a width of at most 3 s, preferably at most 2 s, more preferably at most 1,75 s.

7. Method according to one of the preceding claims, **characterized in that** the analysis windows (10) are overlapping or non-overlapping sliding windows over a measurement time for each channel, preferably that at least two local activation potentials (12) are extracted per channel, in particular at least one local activation potential (12) is extracted per analysis window (10), more preferably that the measurement time is at least 1 s, preferably at least 2,5 s, more preferably at least 10 s.

8. Method according to one of the preceding claims, **characterized in that** the control system (3) carries out the identification routine (9) and the classification routine (15) on a multi-channel intracardiac electrogram (2) of the measurement location (1) recorded after an ablation procedure applied near to, in particular around, the measurement location (1) to determine the cardiac isolation status of the measurement location (1), preferably that additionally the control system (3) carries out at least the identification routine (9) on a multi-channel intracardiac electrogram (2) of the measurement location (1) recorded prior to the ablation procedure and determines the cardiac isolation status based on a comparison of the local activation potentials (12) prior to and after the ablation procedure.

9. Method according to one of the preceding claims, **characterized in that** the activation search algorithm comprises a peak detection algorithm for finding the local activation potentials (12) and subsequently the windows (W) of the local activation potentials (12), preferably that the peak detection algorithm is based on non-linear filters, in particular wavelet filters, and/or a transformation of the electrogram (2), in particular a wavelet transformation, and/or comprises a peak detection by amplitude.

10. Method according to one of the preceding claims, **characterized in that** in the identification routine (9) the control system (3) identifies a fixed number of local activation potentials (12) per channel and/or per analysis window (10), preferably a fixed number per time interval (26), in particular per measurement window, more preferably that the fixed number is based on a physiological and/or measured heart rate, more preferably that the fixed number is a maximum of one local activation potential (12) per at least 500 ms, preferably per at least 800 ms, more preferably per at least 1 s.

11. Method according to one of the preceding claims, **characterized in that** the control system (3) executes an interference signal removal step prior to the identification step, the interference signal removal step comprising complete or weighted blanking of time intervals (26) around and/or relative to pacing artefacts and/or CS-potentials (13) and/or ECG-waves (27), preferably that the pacing artefacts and/or CS-potentials (13) and/or ECG-waves (27) are detected on an electrogram (2) different from the multi-channel intracardiac electrogram (2), the electrogram (2) being a coronary sinus or surface electrogram (2).

12. Method according to one of the preceding claims, **characterized in that** the weighted blanking is an application of different weights (28) to the electrogram (2) inside the respective time interval (26), preferably that the weights (28) are predetermined to comprise a section of complete removal of the respective time interval (26) and at least one section of reducing the amplitude of the electrogram (2).

13. Method according to one of the preceding claims, **characterized in that** the control system (3) executes a quality control step, that in the quality control step the control system (3) removes local activation potentials (12) and/or time sections of electrogram (2) channels (11) and or electrogram (2) channels (11) based on a quality parameter, preferably that the quality parameter is a noise parameter, more preferably a root-mean-square ratio and/or based on a power-line interference detection and/or a peak-to-baseline ratio.

14. Method according to one of the preceding claims, **characterized in that** the multi-channel electrogram (2) comprises at least four, preferably at least six, more preferably at least eight channels (11), and/or, that the multi-channel electrogram (2) was recorded by an ablation catheter (6).

15. Control system configured to perform the method according to one of the preceding claims, wherein the control system (3) is configured to receive and/or measure the multi-channel electrogram (2), preferably wherein the control system (3) is connectable to the ablation catheter (6).

## Patentansprüche

1. Verfahren zur Bestimmung eines Herzisolationszustands einer Messstelle (1) in Gegenwart von Fernfeldinterferenz durch Analysieren eines mehrkanaligen intrakardialen Elektrogramms (2) der Messstelle (1) mittels eines Steuersystems (3),
wobei das Steuersystem (3) in einer Identifizierungsroutine (9) einen Aktivierungssuchalgorithmus auf Analysefenster (10) von mindestens 400 ms in mindestens zwei unterschiedlichen Kanälen (11) des intrakardialen Elektrogramms (2) anwendet, wobei der Aktivierungssuchalgorithmus Fenster (W) von lokalen Aktivierungspotentialen (12) innerhalb der Analysefenster (10) identifiziert,
wobei das Steuersystem (3) in einer Klassifizierungsroutine (15) die lokalen Aktivierungspotentiale (12) analysiert, um den Herzisolationszustand der Messstelle (1) zu bestimmen.

2. Verfahren nach Anspruch 1, **dadurch gekennzeichnet, dass** das intrakardiale Elektrogramm (2) während einer Vorhofarrhythmie aufgezeichnet worden ist, speziell Vorhofflimmern oder Vorhofflattern, und/oder dass die Messstelle (1) sich mindestens teilweise innerhalb eines Vorhofs (16), insbesondere dem linken Vorhof (16) befindet, wobei die Messstelle (1) vorzugsweise eine Insel (17) in der Wand (18) des linken Vorhofs (16) oder am Eingang einer Lungenvene (19) ist, und/oder dass die Messstelle (1) sich mindestens teilweise innerhalb einer Lungenvene (19) befindet.

3. Verfahren nach Anspruch 1 oder 2, **dadurch gekennzeichnet, dass** das Steuersystem (3) in der Klassifizierungsroutine (15) eine Morphologie der lokalen Aktivierungspotentiale (12) analysiert, um den Herzisolationszustand der Messstelle (1) zu bestimmen, wobei das Steuersystem (3) vorzugsweise in der Klassifizierungsroutine (15) die lokalen Aktivierungspotentiale (12) zu Morphologiegruppen (20) klassifiziert und vorzugsweise basierend auf der Verteilung von lokalen Aktivierungspotentialen (12) über die Gruppen den Herzisolationszustand bestimmt.

4. Verfahren nach Anspruch 3, **dadurch gekennzeichnet, dass** das Steuersystem (3) in der Klassifizierungsroutine (15) die lokalen Aktivierungspotentiale (12) basierend auf einer Anzahl von charakteristischen Peaks des lokalen Aktivierungspotentials (12) in Morphologiegruppen (20) klassifiziert, wobei das Steuersystem (3) vorzugsweise einen Peak mit mindestens einer vorbestimmten Amplitude und/oder mit mindestens einer vorbestimmten Neigung und/oder mit höchstens einer vorbestimmten Neigung und/oder mit mindestens einem vorbestimmten minimalen Peak-Abstand und/oder mit höchstens einem vorbestimmten maximalen Peak-Abstand und/oder basierend auf einer Peak-Morphologie, insbesondere einem minimalen und/oder maximalen Peak-Winkel, als charakteristischen Peak bestimmt.

5. Verfahren nach Anspruch 4, **dadurch gekennzeichnet, dass** die Morphologiegruppen (20) eine Gruppe für lokale Aktivierungspotentiale (12) mit einem einzelnen charakteristischen Peak und/oder genau zwei charakteristischen Peaks und/oder genau drei charakteristischen Peaks und/oder mehr als drei charakteristischen Peaks und/oder mindestens zwei charakteristischen Peaks, die durch eine vorbestimmte Zeit getrennt sind, umfassen.

6. Verfahren nach einem der vorhergehenden Ansprüche, **dadurch gekennzeichnet, dass** die Analysefenster (10) eine Breite von mindestens 400 ms, vorzugsweise mindestens 800 ms, bevorzugter mindestens 1,25 s aufweisen, und/oder dass die Analysefenster (10) eine Breite von höchstens 3 s, vorzugsweise höchstens 2 s, bevorzugter höchstens 1,75 s aufweisen.

7. Verfahren nach einem der vorhergehenden Ansprüche, **dadurch gekennzeichnet, dass** die Analysefenster (10) überlappende oder nicht-überlappende gleitende Fenster über eine Messzeit für jeden Kanal sind, vorzugsweise dass mindestens zwei lokale Aktivierungspotentiale (12) pro Kanal extrahiert werden, insbesondere mindestens ein lokales Aktivierungspotential (12) pro Analysefenster (10) extrahiert wird, wobei bevorzugter die Messzeit mindestens 1 s, vorzugsweise mindestens 2,5 s, bevorzugter mindestens 10 s ist.

8. Verfahren nach einem der vorhergehenden Ansprüche, **dadurch gekennzeichnet, dass** das Steuersystem (3) die Identifizierungsroutine (9) und die Klassifizierungsroutine (15) auf einem mehrkanaligen intrakardialen Elektrogramm (2) der Messstelle (1) ausführt, das nach einem Ablationseingriff aufgezeichnet wurde, der nahe, insbesondere um die Messstelle (1) angewendet wird, um den Herzisolationszustand der Messstelle (1) zu bestimmen, wobei vorzugsweise das Steuersystem (3) zudem mindestens die Identifizierungsroutine (9) auf einem mehrkanaligen intrakardialen Elektrogramm (2) der Messstelle (1) ausführt, das vor dem Ablationseingriff aufgezeichnet wurde, und den Herzisolationszustand basierend auf einem Vergleich der lokalen Aktivierungspotentiale (12) vor und nach dem Ablationseingriff bestimmt.

9. Verfahren nach einem der vorhergehenden Ansprüche, **dadurch gekennzeichnet, dass** der Aktivierungssuchalgorithmus einen Peakerkennungsalgorithmus zum Finden der lokalen Aktivierungspotentiale (12) und nachfolgend der Fenster (W) der lokalen Aktivierungspotentiale (12) umfasst, wobei vorzugsweise der Peakerkennungsalgorithmus auf nicht-linearen Filtern, insbesondere Wavelet-Filtern, und/oder einer Transformation des Elektrogramms (2), insbesondere einer Wavelet-Transformation, basiert und/oder eine Peakerkennung gemäß Amplitude umfasst.

10. Verfahren nach einem der vorhergehenden Ansprüche, **dadurch gekennzeichnet, dass** das Steuersystem (3) in der Identifizierungsroutine (9) eine feste Anzahl lokaler Aktivierungspotentiale (12) pro Kanal und/oder pro Analysefenster (10), vorzugsweise eine feste Anzahl pro Zeitintervall (26), insbesondere pro Messfenster identifiziert, wobei die feste Anzahl bevorzugter auf einer physiologischen und/oder gemessenen Herzfrequenz basiert, wobei bevorzugter die feste Anzahl ein Maximum eines lokalen Aktivierungspotentials (12) pro mindestens 500 ms, vorzugsweise pro mindestens 800 ms, bevorzugter pro mindestens 1 s ist.

11. Verfahren nach einem der vorhergehenden Ansprüche, **dadurch gekennzeichnet, dass** das Steuersystem (3) einen Interferenzsignalentfernungsschritt vor dem Identifizierungsschritt ausführt, wobei der Interferenzsignalentfernungsschritt vollständiges oder gewichtetes Austasten von Zeitintervallen (26) um und/oder relativ zu Stimulatorartefakten und/oder CS-Potentialen (13) und/oder EKG-Wellen (27) umfasst, wobei vorzugsweise die Stimulatorartefakte und/oder CS-Potentiale (13) und/oder EKG-Wellen (27) auf einem Elektrogramm (2) erkannt werden, das sich von dem mehrkanaligen intrakardialen Elektrogramm (2) unterscheidet, wobei das Elektrogramm (2) ein Koronarsinus- oder Oberflächenelektrogramm (2) ist.

12. Verfahren nach einem der vorhergehenden Ansprüche, **dadurch gekennzeichnet, dass** das gewichtete Austasten eine Anwendung von unterschiedlichen Gewichtungen (28) auf das Elektrogramm (2) innerhalb des jeweiligen Zeitintervalls (26) ist, wobei vorzugsweise die Gewichtungen (28) vorbestimmt werden, so dass sie einen Abschnitt mit vollständiger Entfernung des jeweiligen Zeitintervalls (26) und mindestens einen Abschnitt des Reduzierens der Amplitude des Elektrogramms (2) umfassen.

13. Verfahren nach einem der vorhergehenden Ansprüche, **dadurch gekennzeichnet, dass** das Steuersystem (3) einen Qualitätskontrollschritt ausführt, wobei das Steuersystem (3) in dem Qualitätskontrollschritt lokale Aktivierungspotentiale (12) und/oder Zeitabschnitte von Kanälen (11) des Elektrogramms (2) und/oder Kanäle (11) des Elektrogramms (2) basierend auf einem Qualitätsparameter entfernt, wobei der Qualitätsparameter vorzugsweise ein Rauschparameter, bevorzugter ein Effektivwertverhältnis ist und/oder auf einer Erkennung von Stromleitungsinterferenz und/oder einem Verhältnis von Peak zu Basislinie basiert.

14. Verfahren nach einem der vorhergehenden Ansprüche, **dadurch gekennzeichnet, dass** das mehrkanalige Elektrogramm (2) mindestens vier, vorzugsweise mindestens sechs, bevorzugter mindestens acht Kanäle (11) umfasst, und/oder dass das mehrkanalige Elektrogramm (2) durch einen Ablationskatheter (6) aufgezeichnet wurde.

15. Steuersystem, das ausgestaltet ist, um das Verfahren gemäß einem der vorhergehenden Ansprüche durchzuführen, wobei das Steuersystem (3) ausgestaltet ist, um das mehrkanalige Elektrogramm (2) zu empfangen und/oder zu messen, wobei das Steuersystem (3) vorzugsweise mit dem Ablationskatheter (6) verbunden werden kann.

## Revendications

1. Procédé de détermination d'un état d'isolation cardiaque d'un lieu de mesure (1) en présence d'interférences en champ lointain par analyse d'un électrogramme intracardiaque multicanal (2) du lieu de mesure (1) par l'intermédiaire d'un système de commande (3),
dans une routine d'identification (9), le système de commande (3) appliquant un algorithme de recherche d'activation à des fenêtres d'analyse (10) d'au moins 400 ms dans au moins deux canaux différents (11) de l'électrogramme intracardiaque (2), l'algorithme de recherche d'activation identifiant des fenêtres (W) de potentiels d'activation locaux (12) à l'intérieur des fenêtres d'analyse (10),
dans une routine de classification (15), le système de commande (3) analysant les potentiels d'activation locaux (12) pour déterminer l'état d'isolation cardiaque du lieu de mesure (1).

2. Procédé selon la revendication 1, **caractérisé en ce que** l'électrogramme intracardiaque (2) a été enregistré pendant une arythmie auriculaire, en particulier une fibrillation auriculaire ou un flutter auriculaire, et/ou, **en ce que** le lieu de mesure (1) est au moins partiellement situé à l'intérieur d'une oreillette (16), en particulier l'oreillette gauche (16), de préférence **en ce que** le point de mesure (1) est un îlot (17) dans la paroi (18) de l'oreillette gauche (16) ou à l'entrée d'une veine pulmonaire (19), et/ou **en ce que** le point de mesure (1) est au moins partiellement situé à l'intérieur d'une veine pulmonaire (19).

3. Procédé selon la revendication 1 ou 2, **caractérisé en ce que**, dans la routine de classification (15), le système de commande (3) analyse une morphologie des potentiels d'activation locaux (12) pour déterminer l'état d'isolation cardiaque du lieu de mesure (1), de préférence **en ce que**, dans la routine de classification (15), le système de commande (3) classe les potentiels d'activation locaux (12) en groupes de morphologie (20) et, de préférence, détermine, sur la base de la distribution des potentiels d'activation locaux (12) à travers les groupes, l'état d'isolation cardiaque.

4. Procédé selon la revendication 3, **caractérisé en ce que**, dans la routine de classification (15), le système de commande (3) classe les potentiels d'activation locaux (12) en groupes de morphologie (20) sur la base d'un nombre de pics caractéristiques du potentiel d'activation local (12), de préférence, le système de commande (3) classe comme pic caractéristique un pic ayant au moins une amplitude prédéterminée et/ou au moins une pente prédéterminée et/ou au plus une pente prédéterminée et/ou au moins une distance de pic minimale prédéterminée et/ou au plus une distance de pic maximale prédéterminée et/ou sur la base d'une morphologie de pic, en particulier d'un angle de pic minimal et/ou maximal.

5. Procédé selon la revendication 4, **caractérisé en ce que** les groupes de morphologie (20) comprennent un groupe pour les potentiels d'activation locaux (12) avec un seul pic caractéristique et/ou exactement deux pics caractéristiques et/ou exactement trois pics caractéristiques et/ou plus de trois pics caractéristiques et/ou au moins deux pics caractéristiques séparés par un temps prédéterminé.

6. Procédé selon l'une quelconque des revendications précédentes, **caractérisé en ce que** les fenêtres d'analyse (10) ont une largeur d'au moins 400 ms, de préférence d'au moins 800 ms, plus préférablement d'au moins 1,25 s, et/ou **en ce que** les fenêtres d'analyse (10) ont une largeur d'au plus 3 s, de préférence d'au plus 2 s, plus préférablement d'au plus 1,75 s.

7. Procédé selon l'une quelconque des revendications précédentes, **caractérisé en ce que** les fenêtres d'analyse (10) sont des fenêtres glissantes se chevauchant ou non sur un temps de mesure pour chaque canal, de préférence **en ce qu'**au moins deux potentiels d'activation locaux (12) sont extraits par canal, en particulier au moins un potentiel d'activation local (12) est extrait par fenêtre d'analyse (10), plus préférablement **en ce que** le temps de mesure est d'au moins 1 s, de préférence d'au moins 2,5 s, plus préférablement d'au moins 10 s.

8. Procédé selon l'une quelconque des revendications précédentes, **caractérisé en ce que** le système de commande (3) exécute la routine d'identification (9) et la routine de classification (15) sur un électrogramme intracardiaque multicanal (2) du lieu de mesure (1) enregistré après une procédure d'ablation appliquée à proximité, en particulier autour, du lieu de mesure (1) pour déterminer l'état d'isolation cardiaque du lieu de mesure (1), de préférence, le système de commande (3) exécute en outre au moins la routine d'identification (9) sur un électrogramme intracardiaque multicanal (2) du point de mesure (1) enregistré avant la procédure d'ablation et détermine l'état d'isolation cardiaque sur la base d'une comparaison des potentiels d'activation locaux (12) avant et après la procédure d'ablation.

9. Procédé selon l'une quelconque des revendications précédentes, **caractérisé en ce que** l'algorithme de recherche d'activation comprend un algorithme de détection de pic pour trouver les potentiels d'activation locaux (12) et ensuite les fenêtres (W) des potentiels d'activation locaux (12), de préférence **en ce que** l'algorithme de détection de pic est basé sur des filtres non linéaires, en particulier des filtres à ondelettes, et/ou une transformation de l'électrogramme (2), en particulier une transformation à ondelettes, et/ou comprend une détection de pic par l'amplitude.

10. Procédé selon l'une quelconque des revendications précédentes, **caractérisé en ce que**, dans la routine d'identification (9), le système de commande (3) identifie un nombre fixe de potentiels d'activation locaux (12) par canal et/ou par fenêtre d'analyse (10), de préférence un nombre fixe par intervalle de temps (26), en particulier par fenêtre de mesure, de préférence **en ce que** le nombre fixe est basé sur une fréquence cardiaque physiologique et/ou mesurée, de préférence **en ce que** le nombre fixe est un maximum d'un potentiel d'activation local (12) par au moins 500 ms, de préférence par au moins 800 ms, plus préférablement par au moins 1 s.

11. Procédé selon l'une quelconque des revendications précédentes, **caractérisé en ce que** le système de commande (3) exécute une étape d'élimination des signaux d'interférence avant l'étape d'identification, l'étape d'élimination des signaux d'interférence comprenant la suppression complète ou pondérée des intervalles de temps (26) autour et/ou par rapport aux artefacts de stimulation et/ou aux potentiels CS (13) et/ou aux ondes ECG (27), de préférence **en ce que** les artefacts de stimulation et/ou les potentiels CS (13) et/ou les ondes ECG (27) sont détectés sur un électrogramme (2) différent de l'électrogramme intracardiaque multicanal (2), l'électrogramme (2) étant un électrogramme de sinus coronaire ou de surface (2).

12. Procédé selon l'une quelconque des revendications précédentes, **caractérisé en ce que** la suppression pondérée est une application de différents poids (28) à l'électrogramme (2) à l'intérieur de l'intervalle de temps respectif (26), de préférence **en ce que** les poids (28) sont prédéterminés pour comprendre une section de suppression complète de l'intervalle de temps respectif (26) et au moins une section de réduction de l'amplitude de l'électrogramme (2).

13. Procédé selon l'une quelconque des revendications précédentes, **caractérisé en ce que** le système de commande (3) exécute une étape de commande de qualité, **en ce que** dans l'étape de commande de qualité le système de commande (3) supprime des potentiels d'activation locaux (12) et/ou des sections temporelles de canaux (11) d'électrogrammes (2) et ou des canaux (11) d'électrogrammes (2) sur la base d'un paramètre de qualité, de préférence **en ce que** le paramètre de qualité est un paramètre de bruit, plus préférablement un rapport moyenne quadratique et/ou sur la base d'une détection d'interférences de ligne d'alimentation et/ou d'un rapport pic-base.

14. Procédé selon l'une quelconque des revendications précédentes, **caractérisé en ce que** l'électrogramme multicanal (2) comprend au moins quatre, de préférence au moins six, plus préférablement au moins huit canaux (11), et/ou, **en ce que** l'électrogramme multicanal (2) a été enregistré par un cathéter d'ablation (6).

15. Système de commande configuré pour mettre en œuvre le procédé selon l'une quelconque des revendications précédentes, le système de commande (3) étant configuré pour recevoir et/ou mesurer l'électrogramme multicanal (2), de préférence le système de commande (3) pouvant être connecté au cathéter d'ablation (6).
